## Europäisches Patentamt

## European Patent Office

## Office européen des brevets

(11) Publication number: **0 067 029**
**B1**

## EUROPEAN PATENT SPECIFICATION

(45) Date of publication of patent specification: 30.04.86

(51) Int. Cl.⁴: **A 61 K 37/14, C 08 G 65/32**

(21) Application number: **82302826.1**

(22) Date of filing: **02.06.82**

(54) Oxygen carrier.

(30) Priority: **10.06.81 JP 89315/81**

(43) Date of publication of application:
**15.12.82 Bulletin 82/50**

(45) Publication of the grant of the patent:
**30.04.86 Bulletin 86/18**

(84) Designated Contracting States:
**DE FR GB**

(56) References cited:
**GB-A-2 055 868**
**US-A-4 136 093**

(73) Proprietor: **AJINOMOTO CO., INC.**
**5-8, Kyobashi 1-chome, Chuo-ku**
**Tokyo 104 (JP)**

(72) Inventor: **Iwashita, Yuji**
**No. 1212 Ichinotsubo Apartment, Nakahara-ku**
**Kawasaki-shi Kanagawa-ken (JP)**
Inventor: **Iwasaki, Keiji**
**No. 2-20-8, Kannon Kawakaki-k**
**Kawasaki-shi Kanagawa-ken (JP)**
Inventor: **Ajisaka, Katsumi**
**No. 1293-4, Nakano-cho Totsuka-ku**
**Yokohama-shi Kanagawa-ken (JP)**

(74) Representative: **Bond, Bentley George et al**
**HASELTINE LAKE & CO. Hazlitt House 28**
**Southampton Buildings Chancery Lane**
**London, WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 0 067 029

## Description

The present invention relates to a modified haemoglobin for use as an oxygen carrier in a blood substitute.

It is known that haemoglobin convalently bonded to a polymer selected from polyethylene glycol, polypropylene glycol and copolymers of ethylene oxide and propylene oxide, is useful as an oxygen carrier for a blood substitute (see JP—12308/1981—A and US—4301144—A).

According to the present invention, there is provided an oxygen carrier comprising haemoglobin or a haemoglobin derivative covalently bonded to a polymer selected from polyethylene glycol, polypropylene glycol, a copolymer of ethylene oxide and propylene oxide, or such a polymer in which at least one hydroxyl group thereof is (a) etherified by an alcohol having from 1 to 16 carbon atoms, (b) esterified by a carboxylic acid having from 2 to 18 carbon atoms, or (c) aminated by an amine having from 1 to 18 carbon atoms, characterised in that the haemoglobin or haemoglobin derivative is covalently bonded to said polymer via an amide bond.

The present invention also provides a method of preparing an oxygen carrier, which comprises introducing at least one carboxyl group onto a polymer by reaction of the polymer with a dicarboxylic acid or a monohalogenated carboxylic acid, and covalently bonding the polymer to haemoglobin or a haemoglobin derivative by reaction of an amino group of the haemoglobin or haemoglobin derivative with the carboxyl group of the polymer to form an amide bond.

The improvement in accordance with the invention is that the haemoglobin or haemoglobin derivative is attached to the polymer by an amide bond.

The polymer used in the present invention is, for example, (a) a polyalkylene glycol (i.e. a polyether) selected from polyethylene glycol, polypropylene glycol and copolymers of ethylene oxide and propylene oxide; (b) an ether of one of the foregoing polyalkylene glycols and an alcohol having from 1 to 16 carbon atoms (examples of such ethers being the monomethyl, monocetyl and monooleyl ethers); (c) an ester of one of the foregoing polyalkylene glycols and a carboxylic acid having from 2 to 18 carbon atoms (examples of such esters being the monobutyl and monostearyl esters); and (d) an amine obtained by reaction of a polyalkylene glycol and an amine having from 1 to 18 carbon atoms (example of such amines being propylamine and stearylamine). The molecular weight of the polymer, e.g. polyether, is preferably from 300 to 20000, more preferably from 750 to 10000, from the point of view of efficiency and viscosity.

As a process for introducing carboxylic groups into the polymer, there can be employed, for example, (a) the known method described in US—4179337—A and US—3941710—A, and (b) the method which comprises attaching at least one carboxylic acid or polycarboxylic acid (such as an alkane dicarboxylic acid) to a terminal hydroxyl group of the polyether to give an ester.

In order to react the polymer into which the carboxyl group has been introduced with haemoglobin or a derivative thereof, there can be employed, for example, (a) a method which comprises converting the polymer into a conventional peptide synthesis product (such as by reaction with N-hydroxysuccinimide, N-hydroxyphthalimide, p-nitrophenol or pentachlorophenol) and reacting the product with haemoglobin or a derivative thereof so as to form an amide bond, or (b) a method which comprises treating the carboxylated polyether with a halogenating agent such as thionyl chloride to give an acid halide of the polymer having at least one carboxyl group and then combining this product with haemoglobin or a derivative thereof.

Examples of the polycarboxylic acids mentioned above are malonic acid, succinic acid, glutaric acid, adipic acid, malic acid and citric acid. The oxgyen carrying ability of the haemoglobin-polymer complexes of the present invention are not reduced by the presence of carboxylic groups in the complex.

The haemoglobin used in the present invention includes haemoglobin obtained from animals such as cattle, swine, sheep, horses, dogs, monkeys and chickens, as well as from human beings, and further includes haemoglobin derivatives such as the pyridoxal-5'-phosphate derivative, the pyridoxal-5'-sulphate derivative, the 2-nor-2-formyl pyridoxal-5'-phosphate derivative, the 2,3-diphosphoglyceric acid derivative, the inositol hexaphosphate derivative, the inositol pentaphosphate derivative, or a derivative derived from a sugar containing a carboxylic or phosphate group.

In the haemoglobin-polymer complexes of the present invention, the number of the polymer attached (substitution degree) and molecular weight of a given complex can be determined by the method of Ajisaka et al (K. Ajisaka and Y. Iwashita, Biochem, Biophys. Res. Commun. 97, 1076—1081, 1981). Thus, for example, from 1 to 20 molecules of polyether carboxylic acid are attached to a given haemoglobin (subunit).

The haemoglobin-polymer comples is preferably prepared according to one of the following methods (1), (2) and (3).

(1) The polyether carboxylic acid and from 1 to 10 mol, preferably 2 mol, of N-hydroxysuccinimide are dissolved in N,N-dimethylformamide in the presence of from 1 to 10 mol, preferably 2 mol, of dicyclohexylcarbodiimide, and reacted for 3 to 20 hours, preferably 8 to 14 hours, at room temperature or with heating. The dicyclohexylurea which precipitates is removed by filtration. By adding ethyl ether to the filtrate an activated ester is obtained. The ester is allowed to react with about 1 to 1/100 mol, preferably 1/5 to 1/30 mol, of haemoglobin or haemoglobin derivative at a pH of 6.5 to 9.5, preferably 7 to 8.5, in an aqueous solution or in a buffer solution. In the first reaction, N-hydroxyphthalimide, p-nitrophenol or

2

pentachlorophenol may be used in place of N-hydroxysuccinimide, whereby almost the same results are obtained.

(2) The polyether carboxylic acid and from 1 to 10 mol, preferably 5 mol, of imidizole are disolved in N,N-dimethylformamide. To this mixture, from 1 to 10 mol, preferably 5 mol, of dicyclohexylcarbodiimide are added, and the mixture is reacted for 5 to 20 hours, preferably 10 hours, under reflux. After the mixture has cooled, the resulting dicyclohexylurea is removed by filtration. By adding ethyl ether to the filtrate, an activated polyether carboxylic acid ester is obtained. This activated polyether carboxylic acid ester is allowed to react with about 1 to 1/100 mol, preferably 1/5 to 1/30 mol, of haemoglobin or haemoglobin derivative at a pH of 7 to 9, preferably 7.5 to 8.5, in an aqueous solution or in a buffer solution. In the second reaction, succinimide or phthalimide may be used in place of imidazole, whereby almost the same results are obtained. In order to activate the polyether carboxylic acid, it may be allowed to react with 1 to 10 mol, preferably 2 mol, of carbonyldiimidazole in N,N-dimethylformamide.

(3) The polyether carboxylic acid is reacted with an excess of thionyl chloride for 1 to 5 hours, preferably 1.5 to 2 hours, at a temperature of 60 to 90°C, preferably 75 to 80°C. Thionyl chloride is removed by distillation under reduced pressure, and the resulting polyether carboxylic acid halide is allowed to react with 1/10 to 1/100 mol, preferably 1/30 to 1/50 mol, of haemoglobin or haemoglobin derivative at a pH of 8.5 to 9.5, in an aqueous solution or in a buffer solution. In the first reaction, another acid halogenating agent such as phosphorus oxychloride or phosphorus pentachloride may be used in place of thionyl chloride.

The safety of the modified haemoglobin of the present invention has been proved by exchange transfusion experiments. Thus, when more than 90% of the blood of rats was exchange transfused with a 6% solution of a modified haemoglobin of the present invention, the rats survived, whereas, when the blood of rats was exchange transfused with 6% bovine serum albumin solution, the rats died before the exchange ratio reached 82%. This result proves the absence of any acute toxicity of the modified haemoglobin of the present invention, whose $LD_{50}$ is estimated to be over 4.5 g/kg.

The present invention is illustrated by the following Examples and Experiments.

Example 1

5 g (0.001 mol) of monomethoxy polyethylene glycol succinate having a mean molecular weight of 5000 and 0.23 g (0.002 mol) of dicyclohexylcarbodiimide were dissolved in 300 ml of N,N-dimethylformamide. The mixture was stirred overnight at room temperature.

The dicyclohexylurea which precipitated was separated by filtration. To the filtrate, 600 ml of ethyl ether were added, whereby monomethoxy polyethylene glycol mono(succinimidyl succinate) separated in a crystalline form. The crystals were separated by filtration and washed with ethyl ether, whereby 4.6 g of white crystalline material were obtained.

0.5 g (0.1 millimol) of the activated polyether ester was added at 0°C to a solution obtained by dissolving 0.5 g (0.0077 millimol) of the haemoglobin derivative of pyridoxal-5'-phosphate in 100 ml of phosphate buffer solution of pH 8.5. The mixture was stirred for 4 hours at 0°C. The product was purified by repeated ultrafiltration using a membrane whose molecular weight cut-off point was 30000 daltons, whereby unreacted activated ester and decomposed materials were removed, and a solution of a modified haemoglobin was obtained. The modified haemoglobin solution had a single peak when subjected to high speed liquid chromatography using a TSK G3000 SW column (Toyo Soda Co., Inc. Japan).

The solution was freeze-dried, whereby 0.58 g of modified haemoglobin was obtained. This modified haemoglobin had a substitution degree of 6.0 and a molecular weight of 95000. The substitution degree and molecular weight were estimated assuming that the modified haemoglobin had formed a tetramer $(\alpha_2\beta_2)$.

Example 2

2 g (1 millimol) of polyethylene glycol adipate having a mean molecular weight of 2000, 0.27 g (1 millimol) of pentachlorophenol and 0.25 g (1.2 millimol) of dicyclohexylcarbodiimide were added to 30 ml of N,N-dimethylformamide, and the mixture was left overnight at room temperature. The dicyclohexylurea which precipitated was filtrated off. The crystalline material which was precipitated by adding ethyl ether to the filtrate was separated by filtration, and was re-crystallized from toluene to give 1.5 g of crystalline material.

0.22 g (0.1 millimol) of the activated ester thus prepared was added slowly to 20 ml of 0.05% aqueous carbonyl-haemoglobin solution. The pH of the solution was kept at 8.5 with 0.2N aqueous sodium hydroxide solution.

The reaction mixture was subjected to repeated ultrafiltration using a membrane whose molecular weight cut-off point was 30000 daltons, whereby 2 ml of a 0.4% modified haemoglobin solution was obtained. The modified haemoglobin had a single peak when subjected to high speed liquid chromatography, and had a substitution degree of 3.5 and a molecular weight of 72000.

Example 3

8.5 g (0.015 mol) of polyethylene glycol monocarboxymethyl ether having a mean molecular weight of 850, 2.07 g (0.015 mol) of p-nitrophenol and 2.3 g (0.015 mol) of dicyclohexylcarbodiimide were dissolved in 300 ml of N,N-dimethylformamide, and reacted overnight at room temperature.

3

Dicyclohexylurea was separated by filtration, and 600 ml of ethyl ether were poured into the filtrate to form 6.2 g of crystalline material.

The activated ester thus prepared was reacted with 10 ml of the haemoglobin derivative of glucose-6-phosphate (1% solution) in the same manner as in Example 1, whereby 10.5 ml of a 0.8% modified haemoglobin solution were obtained. The modified haemoglobin has a substitution degree of 6.2 and a molecular weight of 70000.

Example 4

4 g (0.002 mol) of monomethoxy polyethylene glycol succinate (molecular weight 2000) and 0.7 g (0.004 mol) of N,N-carbonyldiimidazole were dissolved in 100 ml of N,N-dimethylformamide and allowed to react overnight at room temperature. 200 ml of ethyl ether were poured into the reaction mixture and the precipitate was separated by filtration and washed well with ethyl ether to give monomethoxy polyethylene glycol imidazolyl succinate (yield: 2.2 g).

6 ml (0.0055 millimol) of 6% solution of the haemoglobin derivative of pyridoxal-5'-phosphate were added to 60 ml of 0.1M Tris buffer solution of pH 8.0, and to the solution, 1 g (0.5 millimol) of the above imide was added.

After 4 hours reaction in an ice bath, the product was subjected to repeated ultrafiltration using a membrane whose molecular weight cut-off point was 30000 daltons, whereby 3.6 ml of an 8.3% aqueous modified haemoglobin solution were obtained. The modified haemoglobin had a substitution degree of 10.4 and a molecular weight of 86000.

Example 5

5 g (0.001 mol) of monomethoxy polyethylene glycol succinate (molecular weight 5000), 0.5 g (0.005 g mol) of succinimide and 1 g (0.005 mol) of dicyclohexylcarbodiimide were dissolved in 50 ml of N,N-dimethylformamide, and the reaction mixture was refluxed for 12 hours.

The dicyclohexylurea which precipitated was filtrated off. To the filtrate, 150 ml of ethyl ether was added, and the resulting precipitate was separated by filtration. The precipitate was washed well with ethyl ether and dried to give 3.2 g of crystalline material.

3.8 ml of a 4.8% modified haemoglobin solution was obtained by the reaction of the above imide with 7 ml of the haemoglobin derivative of pyridoxal-5'-phosphate (concentration 3.2%) by the same procedure as in Example 4. The modified haemoglobin had a substitution degree of 11.2 and a molecular weight of 120000.

Example 6

5 g (0.001 mol) polyethylene glycol monocarboxymethyl ether having a mean molecular weight of 5200 was dissolved in 20 ml of thionyl chloride and heated for 1.5 hours at a temperature of 75—80°C.

Unreacted thionyl chloride was removed by distillation under reduced pressure. The resulting acid chloride in crystalline form was dried well. 20 ml of a 1% solution of the carbonylhaemoglobin derivative of pyridoxal-5'-phosphate was dissolved in 200 ml of 0.7M borate buffer solution of pH 10.0. To the solution, 5 g of the above acid chloride were added slowly at 0°C.

After 3 hours' stirring at 0°C, the reaction mixture was subjected to repeated ultrafiltration using a membrane whose molecular weight cut-off point was 50000 daltons, whereby 4.6 ml of a 4.1% modified haemoglobin solution was obtained. The modified haemoglobin had a substitution degree of 9.3 and a molecular weight of 114000.

Example 7

100 g (0.025 mol) of polyethylene glycol (molecular weight 4000) and 6.3 g (0.063 mol) of succinic acid anhydride were dissolved in 100 ml of N,N-dimethylformamide. The mixture was stirred for 3 hours at 100°C, and the reaction mixture was then cooled.

To the mixture, 400 ml of ethyl ether were added, and the resulting precipitate was separated by filtration, washed well with ethyl ether, and dried to give 97.5 g of polyethylene glycol disuccinate in the form of a crystalline material.

97.5 g (0.024 mol) of the crystalline material, 6.3 g (0.054 mol) of N-hydroxysuccinimide, and 11.4 g (0.054 mol) of dicyclohexylcarbodiimide were dissolved in 100 ml of N,N-dimethylformamide with heating, and the mixture was then stirred overnight at 30°C. The dicyclohexylurea which precipitated was separated by filtration.

Polyethylene glycol bis(succinimidyl succinate) in a crystalline form, produced by adding 300 ml of ethyl ether to the filtrate, was separated by filtration, washed well with ethyl ether and dried to give 95 g of a white crystalline material.

10 ml (0.025 millimol) of a 16.4% haemoglobin solution was poured into 35 ml of borate buffer solution of pH 8.5. To the mixture, 4.3 g (1.0 millimol) of the crystalline polyethylene glycol bis(succinimidyl succinate) were added. The mixture was stirred overnight at 4°C.

The product was purified by repeated ultrafiltration using a membrane whose molecular weight cut-off point was 100000 dalton, whereby 51 ml of a 3.2% modified haemoglobin aqueous solution were obtained. The modified haemoglobin had a substitution degree of 11.2 and a molecular weight of 122000.

Example 8

14.3 ml (0.1 millimol) of a 15% solution of the haemoglobin derivative of pyridoxal-5'-phosphate was added to 270 ml of phosphate buffer solution of pH 7.0. To this solution, 4.43 g (1.1 millimol) of crystalline polyethylene glycol bis (succinimidyl succinate) were added slowly at 4°C.

The mixture was stirred for 4 hours at 4°C, and then the product was purified by repeated ultrafiltration using a membrane whose molecular weight cut-off point was 100000 daltons, whereby 11 ml of a 11.6% modified haemoglobin solution were obtained. The modified haemoglobin had a substitution degree of 6.6 and a molecular weight of 93000.

Experiment 1

The oxygen dissociation curves of the haemoglobin-polyether complexes prepared in accordance with the above Examples were determined by the method of Imai et al (K. Imai, H. Morimoto, M. Kotani, H. Watari, H. Waka and M. Kuroda, Biochim. Biophys. Acta, *200*, 189—196, 1970), and therefrom the oxygen partial pressure at which half of the haemoglobin is saturated with oxygen ($P_{50}$ value), was estimated. The results are given in Table 1.

By use of the same haemoglobin-polyether complexes, the residence time in the circulatory system of rats was measured as follows. Two rats (weight about 350 g) were used for each measurement. The rats were infused with 5 ml of a 4—6% solution of the haemoglobinpolyether complex per kg of body weight through the femoral vein, and samples of blood, each 0.2 ml, were withdrawn 5, 10, 15, 30, 60, 90 and 120 minutes after the injection. Each blood sample was centrifuged, and the concentration of haemoglobin in the plasma was determined by the cyanomethemoglobin method.

The half residence time for each sample was estimated from a graph of the change in concentration against the time after the injection. The results are given in Table 1.

TABLE 1

| Example No. | $P_{50}$ value** (mm Hg) | Half residence time (minutes) |
|---|---|---|
| 1 | 8.8 | 150 |
| 2* | 3.6 | 120 |
| 3 | 4.3 | 150 |
| 4 | 4.4 | 180 |
| 5 | 9.8 | 250 |
| 6* | 3.1 | 220 |
| 7 | 3.0 | 170 |
| 8 | 7.7 | 160 |
| The haemoglobin polyether complex of Example 2 of US—4301144—A | 13.5 | 120 |
| Haemoglobin, free (control) | 8.7 | 35 |

*: Carbon monooxide was removed under an oxygen stream, and thereafter the $P_{50}$ value and half residence time were determined.

**: 25°C, pH 7.4, 0.1N NaCl

From these results, it can be seen that the half residence times of the haemoglobin-polyether complexes of the Examples of the present invention in the circulatory systems of rats were from 4 to 7 times as long as that of haemoglobin itself. Furthermore, the complexes of the Examples of the present invention have a remarkable ability to deliver oxygen in the tissues of the organs.

Experiment 2

The Bohr Effect of the haemoglobin-polyether complex prepared in accordance with Example 2 of the present invention and in accordance with Example 4 of US—4301144—A, were measured, according to the method of Bucci and Fronticelli (E. Bucci and C. Fronticelli, Method in Enzymology, Vol. 76, 523—533 (1981)). The results are given in Table 2.

TABLE 2

| Sample | Bohr coefficient* |
|---|---|
| Example 5 of this invention | 0.33 |
| Example 4 of US—4301144—A | 0.12 |
| Haemoglobin, free (Control) | 0.48 |

\* 25C, 0.1M phosphate buffer

From the results of Table 2, it can be seen that the Bohr Effect of the modified haemoglobin of Example 5 of the present invention was about 3 times larger than that of the modified haemoglobin of Example 4 of US—4301144—A. Consequently, the former has a superior ability in transporting carbon dioxide from the tissues to the lungs.

Experiment 3

The colloidal osmotic pressures of 6% solutions of the haemoglobin-polyether complexes prepared according to Examples 1, 7 and 8 of this invention and according to Example 6 of US—4301144—A, were measured. The results are given in Table 3.

TABLE 3

| Sample | Colloidal osmotic pressure* (mm Hg) |
|---|---|
| Example 1 of this invention | 39.4 |
| Example 7 of this invention | 42.5 |
| Example 8 of this invention | 38.8 |
| Example 6 of US—4301144—A | 156.6 |
| Blood (control) | 31.0 |

\* The colloidal osmotic pressure was measured by a 4100 Colloid Osmometer (Wescor Inc.) at 25C.

From the above results, it can be seen that the colloidal osmotic pressure of the complexes of Examples 1, 7 and 8 of this invention renders the complexes more suitable for blood transfusion purposes than the complex of Example 6 of US—4301144—A. Thus, the complexes of Examples 1, 7 and 9 of the present invention are superior in their ability to carry carbon dioxide from the tissues to the lungs and in their safety (because of their lower colloidal osmotic pressure). Therefore, the complexes are very useful for oxygen carriers in blood substitutes.

## Claims

1. An oxygen carrier comprising haemoglobin or a haemoglobin derivative covalently bonded to a polymer selected from polyethylene glycol, polypropylene glycol, a copolymer of ethylene oxide and propylene oxide, or such a polymer in which at least one hydroxyl group thereof is (a) etherified by an alcohol having from 1 to 16 carbon atoms, (b) esterified by a carboxylic acid having from 2 to 18 carbon atoms, or (c) aminated by an amine having from 1 to 18 carbon atoms, characterised in that the haemoglobin or haemoglobin derivative is covalently bonded to said polymer via an amide bond.

2. An oxygen carrier as claimed in claim 1, wherein the haemoglobin derivative is haemoglobin modified with pyridoxal or a derivative thereof, or with a sugar phosphate, or with 2,3-phosphoglyceric acid or a derivative thereof. .

3. An oxygen carrier as claimed in claim 2, wherein the derivative is the pyridoxal-5'-phosphate derivative of haemoglobin, the pyridoxal-5'-sulphate derivative of haemoglobin, the 2-nor-2-formyl-pyridoxal-5'-phosphate derivative of haemoglobin, or the glucose-6-phosphate derivative of haemoglobin.

4. An oxygen carrier as claimed in any of claims 1 to 3, wherein said polymer has a molecular weight of from 300 to 20000.

5. An oxygen carrier as claimed in claim 4, wherein said polymer has a molecular weight of from 750 to 10000.

6. A method of preparing an oxygen carrier, which comprises introducing at least one carboxyl group onto a polymer by reaction of the polymer with a dicarboxylic acid or a monohalogenated carboxylic acid, and covalently bonding the polymer to haemoglobin or a haemoglobin derivative by reaction of an amino group of the haemoglobin or haemoglobin derivative with the carboxyl group of the polymer to form an amide bond.

**Patentansprüche**

1. Sauerstoffträger, der Hämoglobin oder ein Hämoglobin-Derivat in kovalenter Bindung an ein Polymeres umfaßt, das ausgewählt ist aus Polyethylenglycol, Polypropylenglycol, einem Copolymeren von Ethylenoxid und Propylenoxid oder einem solchen Polymeren, in dem mindestens eine Hydroxylgruppe (a) mit einem Alkohol mit 1 bis 16 Kohlenstoffatomen verethert, (b) mit einer Carbonsäure mit 2 bis 18 Kohlenstoffatomen verestert oder (c) mit einem Amin mit 1 bis 18 Kohlenstoffatomen aminiert ist, dadurch gekennzeichnet, daß das Hämoglobin oder Hämoglobin-Derivat über eine Amidbindung kovalent an das Polymere gebunden ist.

2. Sauerstoffträger nach Anspruch 1, in dem das Hämoglobin-Derivat Hämoglobin ist, das mit Pyridoxal oder einem Pyridoxal-Derivat, mit einem Zuckerphosphat oder mit 2,3-Phosphoglycerinsäure oder deren Derivat-modifiziert ist.

3. Sauerstoffträger nach Anspruch 2, bei dem das Derivat das Pyridoxyl-5'-phosphatderivat von Hämoglobin, das Pyridoxal-5'-sulfatderivat von Hämoglobin oder das 2-Nor-2-formylpyridoxal-5'-phosphatderivat von Hämoglobin oder das Glucose-6-phosphatderivat von Hämoglobin ist.

4. Sauerstoffträger nach einem der Ansprüche 1 bis 3, bei dem das Polymere ein Molekulargewicht von 300 bis 20 000 hat.

5. Sauerstoffträger nach Anspruch 4, bei dem das Polymere ein Molekulargewicht von 750 bis 10 000 hat.

6. Verfahren zur Herstellung eines Sauerstoffträgers, dadurch gekennzeichnet, daß man durch Umsetzung eines Polymeren mit einer Dicarbonsäure oder einem Carbonsäure-monohalogenid mindestens eine Carboxylgruppe in ein Polymeres einführt und das Polymere durch Umsetzung einer Aminogruppe von Hämoglobin oder eines Hämoglobin-Derivats mit der Carboxylgruppe des Polymeren unter Bildung einer Amidbindung kovalent an Hämoglobin bindet.

**Revendications**

1. Porteur d'oxygène comprenant de l'hémoglobine ou un dérivé d'hémoglobine combiné par covalence à un polymère choisi parmi le polyéthyléneglycol, le polypropylèneglycol, un copolymère d'oxyde d'éthylène et d'oxyde de propylène, ou un tel polymère dans lequel au moins un groupe hydroxyle est (a) éthérifié par un alcool ayant de 1 à 16 atomes de carbone, (b) estérifié par un acide carboxylique ayant de 2 à 18 atomes de carbone, ou (c) aminé par une amine ayant de 1 à 18 atomes de carbone, caractérisé en ce que l'hémoglobine ou le dérivé d'hémoglobine est combiné par covalence audit polymère par une liaison amide.

2. Porteur d'oxygène selon la revendication 1, dans lequel le dérivé d'hémoglobine est l'hémoglobine modifiée avec le pyridoxal ou un dérivé de ce dernier, ou avec un phosphate de sucre, ou avec l'acide 2,3-phosphoglycérique ou un dérivé de ce dernier.

3. Porteur d'oxygène selon la revendication 2, dans lequel le dérivé est le dérivé pyridoxal-5'-phosphate d'hémoglobine, le dérivé pyridoxal-5'-sulfate d'hémoglobine, le dérivé 2-nor-2-formyl-pyridoxal-5'-phosphate d'hémoglobine, ou le dérivé glucose-6-phosphate d'hémoglobine.

4. Porteur d'oxygène selon l'une des revendications 1 à 3, dans lequel ledit polymère a un poids moléculaire compris entre 300 et 20 000.

5. Porteur d'oxygène selon la revendication 4, dans lequel le polymère a un poids moléculaire compris entre 750 et 10 000.

6. Procédé de préparation d'un porteur d'oxygène, qui comprend l'introduction d'au moins un groupe carboxyle dans un polymère par réaction du polymère avec un acide dicarboxylique ou un acide carboxylique monohalogéné et la combinaison par covalence du polymère à l'hémoglobine ou à un dérivé d'hémoglobine par réaction d'un groupe amino de l'hémoglobine ou du dérivé d'hémoglobine avec le groupe carboxyle du polymère pour former une liaison amide.